# DEMANDE DE BREVET EUROPEEN

(11) **EP 1 916 009 A1**
(43) Date de publication de la demande: **30.04.2008**
(21) Numéro de dépôt: 06122902.7
(22) Date de dépôt: 25.10.2006
(51) Int. Cl.: A61M 11/04, A61M 15/00

(54) **Inhalateur à vapeur sèche**

(71) Demandeur: Lenggenhager, Yanis, 2000 Neuchâtel (CH)
(72) Inventeur: Lenggenhager, Yanis, 2000 Neuchâtel (CH)
(74) Mandataire: GLN

(57) **Abrégé**

La présente invention concerne un inhalateur à vapeur sèche, permettant l'extraction des principes actifs de plantes ou huiles, notamment des plantes et huiles médicinales, par chauffage utilisant plus particulièrement comme source de chaleur une flamme.
L'inhalateur comprend un élément de chauffe (1) et un dispositif d'aspiration. L'élément de chauffe (1) comprend un fond (3) et des parois latérales (4) saillant dudit fond (3) et délimitant une chambre d'aspiration (5) communiquant avec le dispositif d'aspiration, le fond (3) de l'élément de chauffe (1) étant traversé par au moins un canal de circulation d'air (6) dont une extrémité (6a) débouche sur les parois latérales (4) de l'élément de chauffe (1) et dont l'autre extrémité (6b) débouche dans la chambre d'aspiration (5).

## Description

### Domaine technique

La présente invention concerne un inhalateur à vapeur sèche, permettant l'extraction des principes actifs de plantes ou huiles, notamment des plantes et huiles médicinales, par chauffage. Plus particulièrement, l'invention concerne un inhalateur à vapeur sèche utilisant comme source de chaleur une flamme provenant par exemple d'un briquet.

### Etat de la technique

Un inhalateur à vapeur sèche, appelé également évaporateur ou vaporiseur, est un appareil avec lequel on peut chauffer des plantes sèches ou fraîches, des huiles ou d'autres préparations à base de plantes voire des médicaments prévus pour être vaporisés, afin d'en extraire le principe actif sous forme de vapeur par apport de chaleur, sans toutefois les brûler. La température à laquelle le principe actif est extrait est comprise entre 30°C et 300°C selon le produit, généralement autour de 180°C. Ainsi, on peut inhaler les substances utiles d'une plante sans absorber d'autres substances non désirées telles que les gaz carboniques et nitriques. Un inhalateur est particulièrement efficace lors d'un traitement thérapeutique et pour un usage en aromathérapie ou en phytothérapie.

Les inhalateurs connus à l'heure actuelle sont, pour la plupart, des appareils de table comprenant des dispositifs de chauffe électriques, qui nécessitent en conséquence une alimentation électrique pour fonctionner. Ces appareils présentent donc l'inconvénient de ne pas être facilement portables. De plus, ils sont de conception sophistiquée et sont donc onéreux. On connait également des inhalateurs de conception plus simple, qui présentent par exemple la forme d'une boule de verre chauffée au moyen d'un briquet. Ce type d'inhalateur est donc particulièrement fragile. Il existe également des inhalateurs, tels que ceux décrits dans la demande de brevet WO 03/082031, dont le fond est traversé par des canaux transversaux. Ces inhalateurs doivent alors comporter des fonds épais pour garantir un bon chauffage de l'air aspiré.

### Divulgation de l'invention

Le but de la présente invention est donc de pallier ces différents inconvénients en proposant un inhalateur à vapeur sèche fonctionnant sans électricité et qui soit de format réduit tout en étant de conception solide et économique, portatif, simple à utiliser, entièrement nettoyable et stérilisable et garantissant un chauffage optimal du produit en évitant toutefois sa combustion.

A cet effet, et conformément à la présente invention, il est proposé un inhalateur à vapeur sèche, comprenant un élément de chauffe et un dispositif d'aspiration, caractérisé en ce que l'élément de chauffe comprend un fond et des parois latérales saillant de ce fond et délimitant une chambre d'aspiration communiquant avec le dispositif d'aspiration, le fond de l'élément de chauffe étant traversé par au moins un canal de circulation d'air dont une extrémité débouche sur les parois latérales de l'élément de chauffe et dont l'autre extrémité débouche dans la chambre d'aspiration.

D'une manière particulièrement avantageuse, le fond de l'élément de chauffe peut comprendre quatre canaux de circulation d'air disposés en croix, leurs extrémités qui débouchent dans la chambre d'aspiration se rassemblant au centre de ladite chambre d'aspiration.

De préférence, les canaux de circulation d'air sont disposés dans le fond de l'élément de chauffe parallèlement audit fond.

Dans un mode de réalisation préféré, les parois latérales de l'élément de chauffe comprennent un prolongement inférieur saillant du fond à l'opposé de la chambre d'aspiration et délimitant avec le fond, un creuset de chauffe.

Afin d'éviter la combustion et d'améliorer la conductivité thermique jusqu'à la substance, les dimensions de l'élément de chauffe sont optimisées. En particulier, l'élément de chauffe est de forme cylindrique et le canal de circulation d'air peut présenter un diamètre inférieur ou égal à 2 mm et être disposé dans le fond de l'élément de chauffe parallèle au creuset de chauffe et à 1 mm ou moins de lui et parallèle à la chambre d'aspiration et à 1 mm ou moins d'elle.

D'une manière avantageuse, le fond de l'élément de chauffe peut comprendre en son centre une forme creuse dans laquelle débouchent les extrémités des canaux de circulation d'air.

De manière à améliorer la conductivité thermique, l'élément de chauffe est réalisé dans un matériau conducteur thermique tel qu'un matériau à base d'aluminium ou un autre matériau présentant une conductivité et une capacité thermiques suffisantes et traité contre la corrosion.

Quant au dispositif d'aspiration, il comprend un cône d'aspiration agencé pour coopérer avec l'élément de chauffe et une paille d'aspiration.

Enfin, on peut prévoir un isolateur thermique disposé autour de l'élément de chauffe et servant de protection.

L'inhalateur selon l'invention est conçu plus particulièrement pour être chauffé au moyen d'une flamme, placée sous le creuset de chauffe.

### Brève description des dessins

D'autres avantages et caractéristiques ressortiront mieux de la description qui va suivre, d'un système conforme à l'invention, donné à titre d'exemple non limitatif, en référence aux dessins annexés sur lesquels:
- la figure 1 est une vue de dessus de l'élément de chauffe de l'inhalateur selon l'invention,
- la figure 2 est une vue en coupe selon la ligne A-A de la figure 1,
- la figure 3 est une vue latérale du dispositif d'aspiration de l'inhalateur selon l'invention, et
- la figure 4 est une vue de dessus du dispositif d'aspiration.

### Mode(s) de réalisation de l'invention

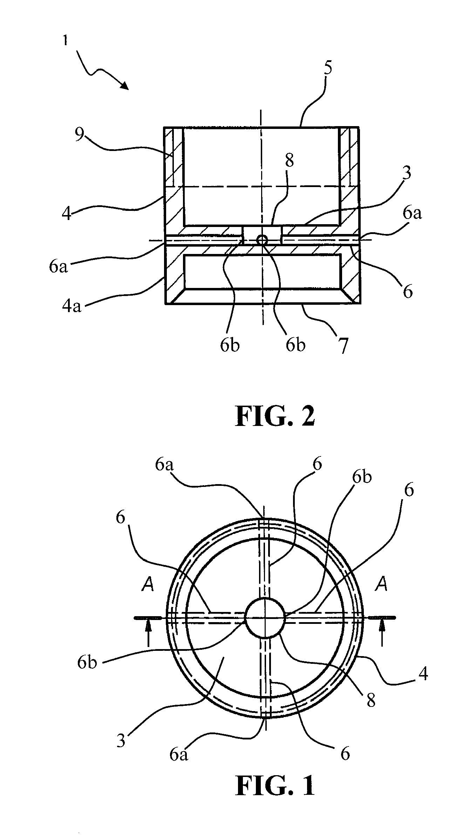

L'inhalateur à vapeur sèche selon l'invention comprend un élément de chauffe 1 et un dispositif d'aspiration 2. En référence aux figures 1 et 2, l'élément de chauffe 1 est de forme cylindrique. Il est réalisé d'une seule pièce en aluminium. Il est bien évident que l'on peut prévoir d'autres formes ou d'autres matériaux conducteurs thermiques sans sortir du cadre de la présente invention. L'élément de chauffe 1 comprend un fond 3 et des parois latérales 4 saillant du fond 3 et délimitant une chambre d'aspiration 5 communiquant avec le dispositif d'aspiration 2.

Les parois latérales 4 de l'élément de chauffe 1 comprennent un prolongement inférieur 4a saillant du fond 3 à l'opposé de la chambre d'aspiration 5 et délimitant avec ledit fond 3 un creuset de chauffe 7.

Selon l'invention, le fond 3 de l'élément de chauffe 1 est traversé par quatre canaux de circulation d'air 6, chacune de leur extrémité 6a débouchant sur les parois latérales 4 de l'élément de chauffe 1 et l'autre extrémité 6b débouchant dans la chambre d'aspiration 5.

Les quatre canaux de circulation d'air 6 sont disposés en croix dans le fond 3 de l'élément de chauffe 5 perpendiculairement aux parois latérales 4, ledit fond 3 comprenant en son centre une forme creuse 8 dans laquelle débouchent et se rassemblent les extrémités 6b des canaux de circulation d'air 6.

Les dimensions de l'élément de chauffe 1 cylindrique optimisées pour éviter la combustion et améliorer la conductivité thermique jusqu'à la substance, sont telles que le diamètre intérieur de l'élément de chauffe est compris entre 10 et 18mm, de préférence sensiblement égal à 16 mm, pour une hauteur comprise entre 10 et 20 mm, de préférence sensiblement égale à 18 mm, et l'épaisseur des parois est comprise entre 1 et 3 mm, de préférence sensiblement égale à 2 mm. Le fond 3 de l'élément de chauffe 1 présente de préférence une épaisseur sensiblement égale à 3 mm et est placé de sorte que le creuset de chauffe 7 a une hauteur de préférence sensiblement égale à 3 mm et la chambre d'aspiration a une hauteur de préférence sensiblement égale à 10 mm. Les canaux de circulation d'air 6 présentent chacun un diamètre de inférieur à 3 mm, de préférence inférieur à 2 mm, avantageusement inférieur ou égal à 1 mm et sont disposés dans le fond 3 de l'élément de chauffe 1, parallèles au creuset de chauffe 7 et à sensiblement 1 mm ou moins de lui et parallèles à la chambre d'aspiration 5 et à sensiblement 1 mm ou moins d'elle. Il est bien évident que les canaux de circulation d'air peuvent aussi être disposés de manière oblique entre les parois latérales 4 et la chambre d'aspiration 5, l'essentiel étant de prévoir pour l'air aspiré un trajet suffisant lorsqu'il traverse l'élément de chauffe de manière à se réchauffer au contact de l'élément de chauffe pour arriver chaud dans la chambre d'aspiration.

L'élément de chauffe 1 peut comprendre une ou plusieurs grilles (non représentées) disposées dans la chambre d'aspiration 5 sur laquelle est placée la substance ou pour répartir des huiles par capillarité. Une grille supplémentaire est disposée à l'entrée du dispositif d'aspiration pour éviter que des particules de l'élément à chauffer ne parviennent à l'utilisateur.

L'élément de chauffe 1 comprend sur ses parois latérales 4 un filetage 9 permettant une fixation le vissage du dispositif d'aspiration 2.

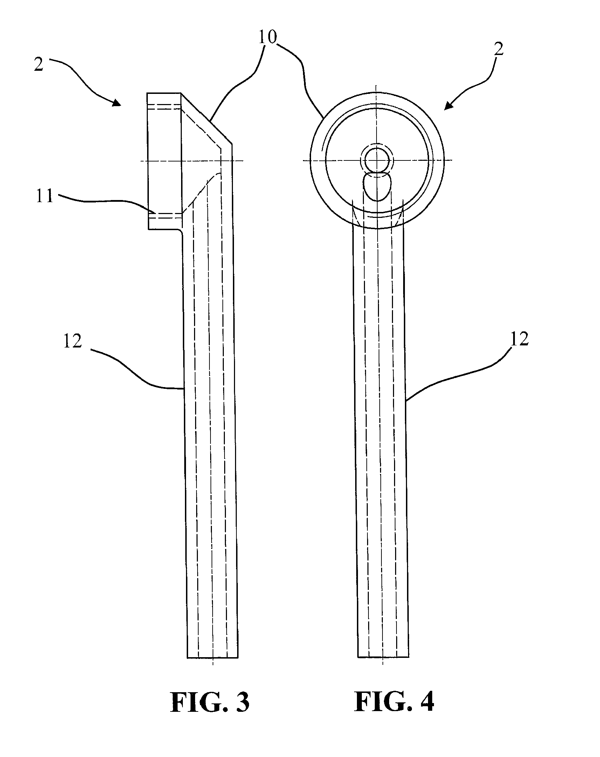

En référence aux figures 3 et 4, le dispositif d'aspiration 2 comprend un cône d'aspiration 10 de dimensions appropriées pour être assemblé à l'élément de chauffe 1. A cet effet, la paroi intérieure du cône d'aspiration 10 comprend un filetage 11 correspondant au filetage 9 de l'élément de chauffe 1 pour assurer l'assemblage des deux pièces par vissage. Le cône d'aspiration 10 permet de compresser la vapeur sèche. Le dispositif d'aspiration 2 comprend également une paille d'aspiration 12 débouchant dans le cône d'aspiration 10. La paille d'aspiration 12 sert également de refroidisseur. Le dispositif d'aspiration 2 est réalisé par exemple en un acier inoxydable médical ou en un autre matériau à usage médical.

Afin de protéger l'utilisateur et d'éviter les brûlures lors de la préhension de l'inhalateur, il peut être prévu un isolateur thermique (non représenté) disposé autour de l'élément de chauffe 1. Cet isolateur thermique peut se présenter sous la forme d'une bague qui vient se fixer sous l'élément de chauffe 1, de manière à ne pas obstruer les extrémités 6a des canaux de circulation d'air 6.

L'inhalateur selon l'invention est d'une conception très solide et économique, permettant une utilisation simple. Ces dimensions réduites en font un inhalateur au format de poche, facilement transportable, tout en étant optimisées pour assurer un temps de chauffe suffisant sans chauffage excessif ou ponctuel et en minimisant le risque de combustion. Les matériaux utilisés permettent un lavage facile et la stérilisation de l'inhalateur.

Lors de l'utilisation de l'inhalateur selon l'invention, la substance est mise en place dans la chambre d'aspiration de l'élément de chauffe, qui est ensuite fermé en vissant le dispositif d'aspiration. Puis l'inhalateur est chauffé au moyen d'une flamme, en utilisant par exemple un briquet avec une flamme dite "chalumeau" ou "laser", ou une autre source de chaleur adaptée, placée sous le creuset de chauffe. La durée du chauffage est d'environ 30 à 60 secondes pour, lors de la première aspiration, amener la substance au niveau de la température d'extraction du principe actif, puis d'environ 10 secondes pour les aspirations suivantes. La forme de l'élément de chauffe et le choix d'un matériau conducteur thermique font que la chaleur diffuse rapidement dans l'élément de chauffe qui fait office de réservoir de chaleur, sans surchauffe ponctuelle. Ensuite, lorsque l'utilisateur aspire de l'air au moyen du dispositif d'aspiration, l'air pénètre dans les différents canaux de circulation d'air. L'air aspiré circule dans les canaux de circulation d'air qui s'étendent entre la paroi latérale et la chambre de combustion, elle-même chauffée par la conduction de chaleur par l'élément de chauffe. L'air aspiré effectue alors un trajet suffisant pour se réchauffer en traversant l'élément de chauffe et arrive donc chaud sur la substance placée dans l'inhalateur, sans la refroidir, ce qui permet d'activer son extraction de manière optimale. L'inertie thermique du matériau de l'élément de chauffe permet une utilisation prolongée et donc plusieurs longues aspirations successives sans procéder à une nouvelle chauffe. On notera que l'élément de chauffe pourrait comporter un nombre inférieur de canaux, mais de diamètre supérieur de manière à conserver un tirage suffisant.

## Revendications

1. Inhalateur à vapeur sèche, comprenant un élément de chauffe (1) et un dispositif d'aspiration (2), **caractérisé en ce que** l'élément de chauffe (1) comprend un fond (3) et des parois latérales (4) saillant dudit fond (3) et délimitant une chambre d'aspiration (5) communiquant avec le dispositif d'aspiration (2), le fond (3) de l'élément de chauffe (1) étant traversé par au moins un canal de circulation d'air (6) dont une extrémité (6a) débouche sur les parois latérales (4) de l'élément de chauffe (1) et dont l'autre extrémité (6b) débouche dans la chambre d'aspiration (5).

2. Inhalateur selon la revendication 1, **caractérisé en ce que** le fond (3) de l'élément de chauffe (1) comprend quatre canaux de circulation d'air (6) disposés en croix, leurs extrémités (6b) qui débouchent dans la chambre d'aspiration (5) se rassemblant au centre de ladite chambre d'aspiration (5).

3. Inhalateur selon l'une quelconque des revendications précédentes, **caractérisé en ce que** les canaux de circulation d'air (6) sont disposés dans le fond (3) de l'élément de chauffe (1) parallèlement audit fond.

4. Inhalateur selon la revendication 1, **caractérisé en ce que** les parois latérales (4) de l'élément de chauffe (1) comprennent un prolongement inférieur (4a) saillant du fond (3) à l'opposé de la chambre d'aspiration (5) et délimitant avec ledit fond (3) un creuset de chauffe (7).

5. Inhalateur selon la revendication précédente, **caractérisé en ce que** le canal de circulation d'air (6) présente un diamètre inférieur ou égal à 2 mm et est disposé dans le fond (3) de l'élément de chauffe (6) parallèle au creuset de chauffe (7) et à 1 mm ou moins de lui et parallèle à la chambre d'aspiration (5) et à 1 mm ou moins d'elle.

6. Inhalateur selon l'une quelconque des revendications précédentes, **caractérisé en ce que** l'élément de chauffe (1) est de forme cylindrique.

7. Inhalateur selon la revendication précédente, **caractérisé en ce que** le fond (3) de l'élément de chauffe (1) comprend en son centre une forme creuse (8) dans laquelle débouchent les extrémités (6b) des canaux de circulation d'air (6).

8. Inhalateur selon l'une quelconque des revendications précédentes, **caractérisé en ce que** l'élément de chauffe (1) est réalisé dans un matériau à base d'aluminium.

9. Inhalateur selon la revendication 1, **caractérisé en ce que** le dispositif d'aspiration (2) comprend un cône d'aspiration (10) agencé pour coopérer avec l'élément de chauffe (1) et une paille d'aspiration (12).

10. Inhalateur selon l'une quelconque des revendications précédentes, **caractérisé en ce qu'**il comprend un isolateur thermique disposé autour de l'élément de chauffe (1).
